# EUROPEAN PATENT APPLICATION

(11) **EP 4 549 639 A1**
(43) Date of publication of application: **07.05.2025**
(21) Application number: 23831507.1
(22) Date of filing: 28.06.2023
(51) Int. Cl.: D01D 5/06, C12M 1/00, D01F 9/04

(54) **GEL FIBER MANUFACTURING APPARATUS AND MANUFACTURING METHOD**

(30) Priority: 29.06.2022 JP 2022104391
(71) Applicant: Mochida Pharmaceutical Co., Ltd., Tokyo 160-8515 (JP)
(72) Inventor: FURUSAKO, Shoji, Tokyo 160-8515 (JP); NARUMI, Tomohiro, Tokyo 160-8515 (JP)
(74) Representative: Hoffmann Eitle
(86) International application number: PCT/JP2023/023968
(87) International publication number: WO 2024/005067

(57) **Abstract**

A gel fiber manufacturing apparatus includes: a first ejection portion configured to eject a main liquid containing a core liquid; a second ejection portion provided to surround a radially outer side of the first ejection portion and configured to eject a sheath fluid that hardens the core liquid; and a sheath fluid supply portion configured to alternately apply to the sheath fluid a constant pressure and a high pressure to supply the sheath fluid to the second ejection portion, the high pressure being higher than the constant pressure.

## Description

### [Technical Field]

The present invention relates to a gel fiber manufacturing apparatus and manufacturing method.

### [Background Art]

Heretofore, gel fibers containing cells and the like have been used in various applications. The technique of Patent Literature 1, for example, has conventionally known as a method and apparatus for manufacturing such a gel fiber.

### [Citation List]

### [Patent Literature]

[PTL 1] Pamphlet of International Publication No. 2020/066306

### [Summary of Invention]

### [Technical Problems]

Like Patent Literature 1, a method has been disclosed as a conventional technique in which a gas is intermittently injected into a gel precursor inside a flow path to form an alternating flow of the gel precursor and the gas, and a gelating agent is caused to join the alternating flow to thereby manufacture gel fibers.

A conventional cutting method as described above is not applicable to elastic and soft gel fibers. Also, while it is possible to employ a method involving cutting a gel fiber with a cutting blade or a method involving cutting a gel fiber by pulling it, it is difficult to neatly cut an elastic and soft gel fiber.

In view of problems as described above, an object of the present invention is to provide a manufacturing apparatus and manufacturing method capable of neatly cutting a gel fiber.

### [Solution to Problems]

An aspect of the present invention is a gel fiber manufacturing apparatus comprising: a first ejection portion configured to eject a main liquid containing a core liquid; a second ejection portion provided to surround a radially outer side of the first ejection portion and configured to eject a sheath fluid that hardens the core liquid; and a sheath fluid supply portion configured to alternately apply to the sheath fluid a constant pressure and a high pressure to supply the sheath fluid to the second ejection portion, the high pressure being higher than the constant pressure.

Another aspect of the present invention is a gel fiber manufacturing method comprising: ejecting a main liquid containing a core liquid; and ejecting a sheath fluid such that the sheath fluid surrounds a radially outer side of the ejected main liquid, the sheath fluid being a fluid which hardens the core liquid; and alternately applying to the sheath fluid a constant pressure and a high pressure that is higher than the constant pressure.

### [Advantageous Effect of Invention]

With the above configurations, it is possible to provide a manufacturing apparatus and manufacturing method capable of neatly cutting a gel fiber.

### [Brief Description of Drawings]

[Fig. 1] Fig. 1 is a diagram schematically illustrating a configuration of a gel fiber manufacturing apparatus according to an embodiment.
[Fig. 2] Fig. 2(a) illustrates a cross-sectional view of nozzles according to the embodiment, and Fig. 2(b) illustrates a cross-sectional view taken along the IIb-IIb line in Fig. 2 (a) .
[Fig. 3] Figs. 3(a), 3(b), and 3(c) are graphs schematically illustrating a time history of a pressure applied to a pressurization liquid by a pump, a time history of a pressure applied to a hardening liquid by a pump, and a time history of a pressure applied to a sheath fluid, respectively.
[Fig. 4] Fig. 4 is a diagram explaining how a generated gel fiber is cut.
[Fig. 5] Fig. 5 is a diagram illustrating states in which gel fibers ejected from the nozzles are preserved.
[Fig. 6] Fig. 6 is a diagram schematically illustrating a configuration of a gel fiber manufacturing apparatus according to a modification.

### [Description of Embodiments]

Embodiments of the present invention will now be described below based on the drawings. Note that "A and/or B" in the following means both A and B, only A, or only B.

### <Embodiment>

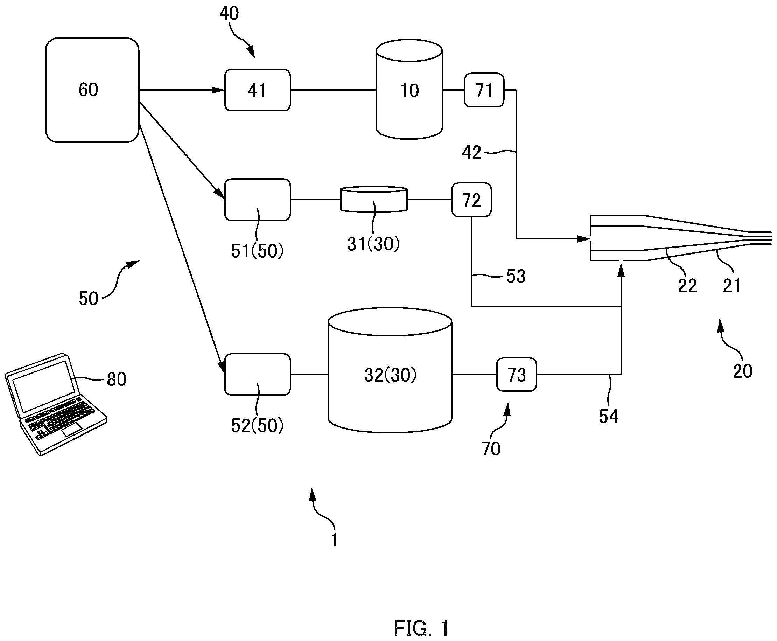

As illustrated in Fig. 1, a gel fiber manufacturing apparatus 1 in an embodiment includes a main liquid storage portion 10 that stores a main liquid as a liquid for forming gel fibers, an ejection portion 20, a sheath fluid storage portion 30, a main liquid supply portion 40, a sheath fluid supply portion 50, a power output unit 60, a measurement device 70, and a controller 80.

The main liquid storage portion 10 is a tank that stores the main liquid which will be a material of gel fibers. The main liquid is a collective term for a core liquid and a central liquid that are formed individually. The core liquid and the central liquid in a state where the core liquid and the central liquid are mixed and in a state where the core liquid and the central liquid are separated will both be referred to collectively as the main liquid herein. The main liquid storage portion 10 in the embodiment uses a single tank to store the main liquid in the state where the core liquid and the central liquid are mixed.

The core liquid is a liquid containing a material of gel fibers, and specific examples thereof include a liquid containing a derivative of alginic acid.

The central liquid is a liquid containing a substance to be contained in gel fibers, and contains cells or a compound. Specific examples of the central liquid containing cells include one in the form of a cell suspension. In the case of using a cell suspension as the central liquid, the gel fibers generated may be referred to as cell-containing gel fibers.

While the cells that may be contained in the central liquid are not particularly limited herein, examples thereof include: cells that produce antibodies (various types of modified antibodies including monoclonal antibodies such as human antibodies, humanized antibodies, chimeric antibodies, and murine antibodies, bispecific antibodies, minibodies, and sugar chain-modified antibodies thereof, and the like); cells that produce biologically active substances (such as enzymes, cytokines, hormones, blood coagulation factors, and vaccines); and cells that are capable of producing various useful substances usable as raw materials for pharmaceuticals, chemicals, food ingredients, and the like. The cells are preferably antibody-producing cells or biologically active substance-producing cells.

Also, while the compound that may be contained in the central liquid is not particularly limited herein, examples thereof include small-molecule drugs, antibody drugs, nucleic acid drugs, peptide drugs, middle-molecule drugs, protein drugs, exosomes.

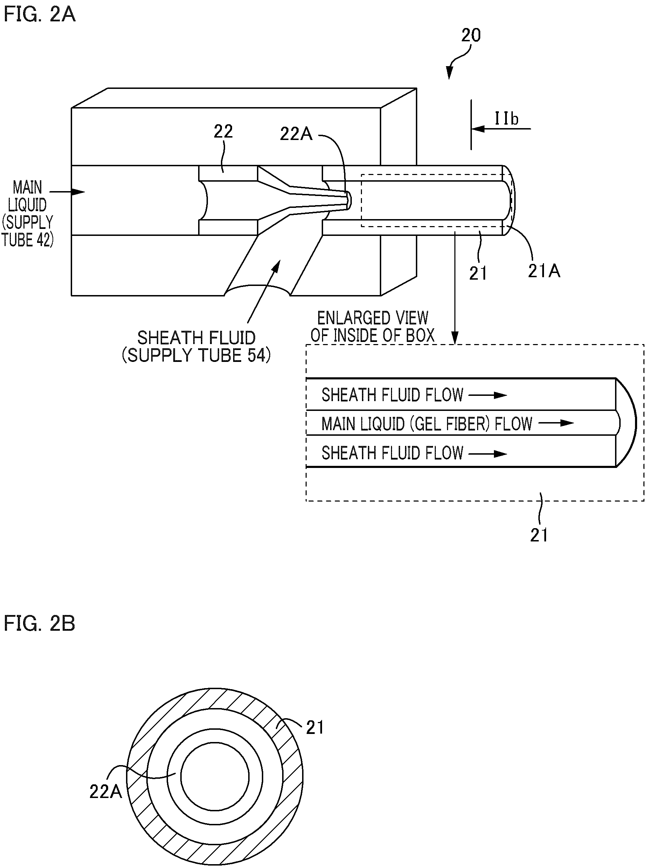

As illustrated in Fig. 2, the ejection portion 20 has two nozzles 21 and 22 disposed coaxially with each other. The nozzle 22 is a tubular member with a substantially circular cross section and has the function of receiving the main liquid supplied from the main liquid supply portion 40 and ejecting the main liquid. Specifically, the nozzle 22 has an ejection tip 22A that is open, and is capable of ejecting the main liquid from the ejection end 22A. While the nozzle 22 is formed in a tapered shape so as to become narrower toward the ejection tip 22A, the nozzle 22 does not necessarily have to become narrower toward 22A as long as the two nozzles 21 and 22 are disposed coaxially with each other.

The nozzle 21 is a tubular or substantially cylindrical member with a substantially circular cross section formed so as to surround a radially outer side of the nozzle 22, and is disposed coaxially with the nozzle 22 (Fig. 2(b)). The nozzle 21 has the function of receiving a sheath fluid supplied from the sheath fluid supply portion 50 and ejecting the sheath fluid. Specifically, the nozzle 21 has an ejection tip 21A that is open in the same direction as the ejection tip 22A, and is capable of ejecting the sheath fluid from the ejection tip 21A. Details of the sheath fluid will be described later.

As illustrated in Fig. 1, the sheath fluid storage portion 30 includes two liquid tanks 31 and 32. The liquid tank 31 is a tank to store a pressurization liquid, and the liquid tank 32 is a tank to store a hardening liquid. The pressurization liquid is a liquid that transmits a pressure to the hardening liquid.

The hardening liquid and the pressurization liquid are each a liquid that forms a part of the sheath fluid. As will be described later in detail, the hardening liquid and the pressurization liquid are mixed in the sheath fluid supply portion 50 to form the sheath fluid. The hardening liquid and the pressurization liquid in a state where the hardening liquid and the pressurization liquid are mixed and in a state where the hardening liquid and the pressurization liquid are separated will both be referred to collectively as the sheath fluid herein.

The sheath fluid and the hardening liquid are liquids that harden the core liquid (and the main liquid containing the core liquid), and their components are determined by the composition of the core liquid. Note that hardening does not necessarily refer herein to solidification but also includes gelation.

The specific combinations of compositions and components contained in the core liquid (or the main liquid) and the hardening liquid (or the sheath fluid) include the following examples. Examples of a solvent to be used to prepare the core liquid or the main liquid include, but are not particularly limited to, culture media, culture media for cell culture, culture liquids, isotonic buffer solutions, phosphate-buffered saline (PBS), tap water, pure water (e.g., distilled water, ion-exchanged water, RO water, RO-EDI water, etc.), ultra-pure water (MilliQ water), saline, and so on. Also, any of the components contained in the core liquid or the main liquid may form a salt, unless specifically stated otherwise.

**[Table 1]**

| Composition(s) contained in core liquid or main liquid | Composition contained in hardening liquid or sheath liquid |
|---|---|
| Alginic acid and/or chemically modified alginic acid | Polyvalent metal ion |
| Fibrin monomer and/or chemically modified fibrin monomer | Calcium ion |
| Collagen and/or chemically modified collagen | Warm water |
| Carrageenan and/or chemically modified carrageenan | Calcium or potassium ion |
| Acrylic acid-based synthetic gel | Sodium ion |
| Chitosan salt and/or chemically modified chitosan salt | Alkaline solution |

A plurality of combination examples among the above may be mixed to prepare the core liquid (or the main liquid) and the hardening liquid (or the sheath fluid). For example, it is possible to employ a liquid containing sodium alginate and a collagen as the core liquid and warm water with polyvalent metal ions as the hardening liquid.

Also, the core liquid may contain a plurality of compositions. For example, a core liquid (or a main liquid) containing both sodium alginate and a carrageenan may be employed. In this case, the composition of the hardening liquid (or the sheath fluid) may contain a corresponding one of the combinations, namely, polyvalent metal ions, calcium ions, or potassium ions.

As the pressurization liquid, the same liquid as the hardening liquid may be used as is, or saline may be used.

As illustrated in Fig. 1, the main liquid supply portion 40 has a pump 41 and a supply tube 42. The supply tube 42 couples the main liquid storage portion 10 and the nozzle 22 and supplies the main liquid to the nozzle 22. The pump 41 has the function of applying a pressure to the main liquid stored in the main liquid storage portion 10 to transport, that is, to send the main liquid to the nozzle 22 through the supply tube 42.

As illustrated in Fig. 1, the sheath fluid supply portion 50 has a supply tube 53, a supply tube 54, and pumps 51 and 52. The supply tube 53 functions as a tube that couples the liquid tank 31 and the supply tube 54 and supplies the pressurization liquid to the supply tube 54. The supply tube 54 functions as a tube that couples the liquid tank 32 and the nozzle 21 and supplies the hardening liquid and the sheath fluid to the nozzle 21.

The pump 52 has the function of applying a pressure to the hardening liquid stored in the liquid tank 32 to send the hardening liquid through the supply tube 54.

Also, the pump 51 has the function of applying a pressure to the pressurization liquid stored in the liquid tank 31 to send the pressurization liquid through the supply tube 53. Since the supply tube 53 is coupled to the supply tube 54, the pressurization liquid thus sent is mixed into the hardening liquid inside the supply tube 54 and thus becomes the sheath fluid. The supply tube 54 supplies the generated sheath fluid to the nozzle 21.

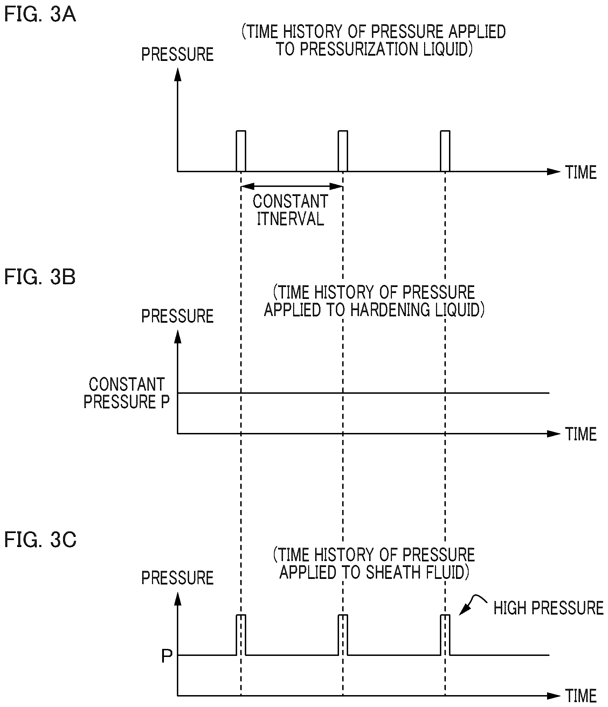

The pumps 41 and 52 apply constant pressures to the insides of the main liquid storage portion 10 and the liquid tank 32 to pressurize the main liquid and the hardening liquid, respectively (Fig. 3(b)). On the other hand, the pump 51 is controlled to apply a pressure to the inside of the liquid tank 31 so as to apply a high pressure to the pressurization liquid at constant intervals, as illustrated in Fig. 3(a). The pressurization liquid is supplied to the inside of the supply tube 54 to transmit the pressure thereon. Thus, as illustrated in Fig. 3(c), the pressure generated on the sheath fluid inside the supply tube 54 shows a time history in which a constant pressure and a regularly generated high pressure appear repetitively.

Note that the magnitudes and intervals of the pressures to be applied to the main liquid, the pressurization liquid, and the hardening liquid by the pumps 41, 51, and 52 are adjusted as appropriate according to the type of gel fibers to be manufactured, the length of a gel fiber, and the like. For example, the intervals of the pressure to be applied by the pump 51 can be irregular according to the length of a gel fiber.
The pressure to be applied to the pressurization liquid is preferably pulse waves. Note that the waveform of the pressure in its time history may be changed as appropriate to a sinusoidal wave, a rectangular wave, a triangular wave, or the like according to the conditions for generating gel fibers.

The power output unit 60 is a device that supplies power to the pumps 41, 51, and 52. In the present embodiment, the power output unit 60 is a compressor that supplies compressed air, and supplies the compressed air as power to each of the pumps 41, 51, and 52. The power output unit 60 may be a device that supplies a different energy such as a device that supplies electricity.

The measurement device 70 is a device that measures the flow rates of fluids, and includes at least three sensors 71, 72, and 73. The sensor 71 is mounted to the supply tube 42 and measures the flow rate of the main liquid flowing through the supply tube 42. The sensor 72 is mounted to the supply tube 53 and measures the flow rate of the pressurization liquid flowing through the supply tube 53. The sensor 73 is mounted to the supply tube 54 and measures the flow rate of the hardening liquid flowing through the supply tube 54.

The controller 80 is communicatively coupled to the measurement device 70, the power output unit 60, the main liquid supply portion 40, and the sheath fluid supply portion 50, and controls operation of the gel fiber manufacturing apparatus 1. Specifically, the controller 80 controls the power output unit 60 and the pumps 41, 51, and 52 based on the flow rates obtained from the measurement device 70.

### (Operation)

The operation of the gel fiber manufacturing apparatus 1 configured as above will now be described.

The controller 80 controls the pumps 51 and 52 so that pressures as illustrated in Fig. 3 can be controlled to be applied to the pressurization liquid and the hardening liquid.

The controller 80 controls the pump 41 so as to apply a constant pressure to the main liquid. The main liquid passes through the nozzle 22 in a state of maintaining a constant speed and flow rate and is ejected from the ejection tip 22A.

The pressurization liquid and the hardening liquid are mixed inside the supply tube 54, thereby forming the sheath fluid, as described above.
The sheath fluid is supplied to the nozzle 21 in a state where pressure is applied thereto as illustrated in Fig. 3(c). As illustrated in "ENLARGED VIEW OF INSIDE OF BOX" in Fig. 2(a), the sheath fluid inside the nozzle 21 flows so as to surround the outer periphery of the main liquid ejected from the ejection tip 22A in the same direction as the main liquid.

The outer periphery of the flow of the main liquid ejected from the ejection tip 22A contacts the sheath fluid and therefore reacts with the sheath fluid and gelates. As a result, the main liquid becomes a gel fiber formed in an elongated fiber shape. The formed gel fiber is ejected from the ejection tip 21A along with the sheath fluid.

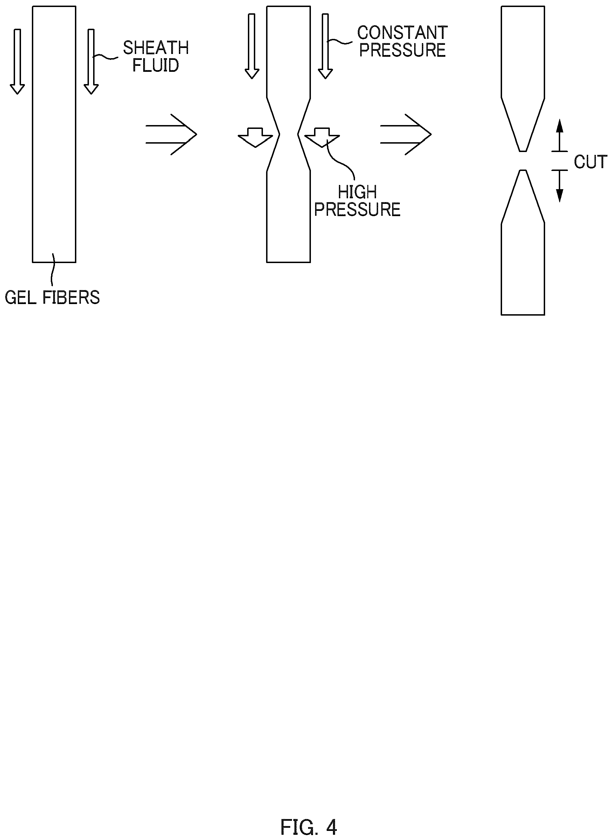

Before being ejected from the ejection tip 21A, the gel fiber is cut at intervals of a constant length by the sheath fluid. Specifically, when a high pressure is applied to the sheath fluid (Fig. 3(c)), the high pressure is also applied to the gel fiber inside the nozzle 21 from radially outside through the sheath fluid. The applied pressure cuts the gel fiber as illustrated in Fig. 4. The high pressure applied to the sheath fluid is generated at constant intervals, so that the gel fiber is cut at intervals of a constant length.

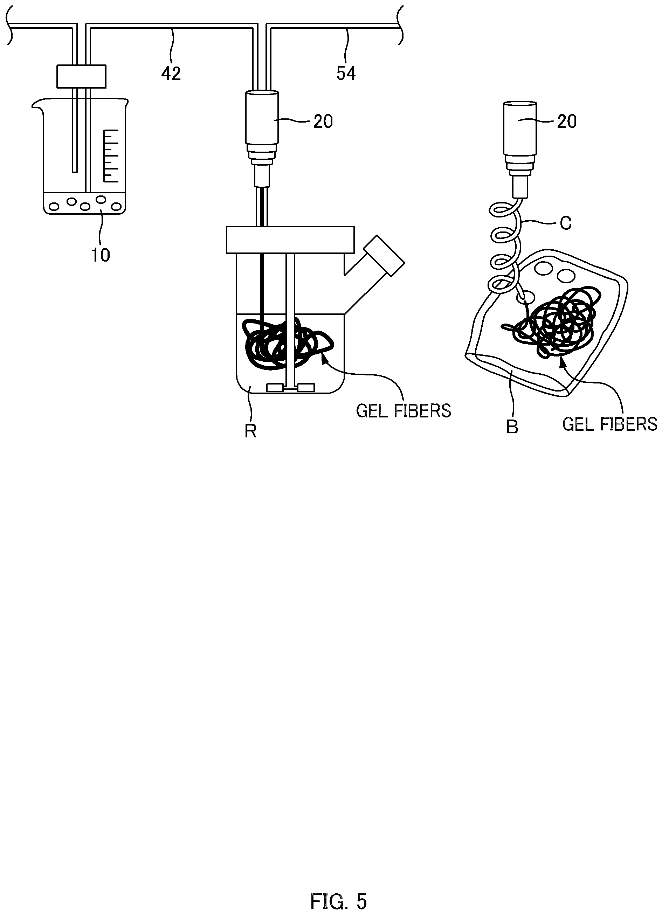

There are various methods for preserving and using the gel fibers ejected from the ejection portion 20. For example, when the gel fibers are manufactured as cell-containing gel fibers, they may be ejected from the ejection portion 20 into a culture liquid in a biological reactor R and preserved therein, as illustrated in Fig. 5. Also, the gel fibers may be ejected through a tube C or the like into a bag B and preserved therein.

### [Example 1]

### (Case of Using Same Composition for Hardening Liquid and Pressurization Liquid)

An example of gel fiber generation using the devices and methods described above will now be described below.

A 3-mass% sodium alginate aqueous solution was prepared. Thereafter, a blue dextran-containing aqueous solution was prepared and mixed with an equal volume of the sodium alginate aqueous solution to generate an alginate-mixed solution as a main liquid. Thereafter, a 100-mM calcium chloride aqueous solution was prepared as a hardening liquid and a pressurization liquid. The main liquid, the pressurization liquid, and the hardening liquid were filled into respective bottles (the main liquid storage portion 10 and the liquid tanks 31 and 32) and corresponding caps were coupled thereto.

One side of each of the supply tubes 42, 53, and 54 (a silicon tube or a PTFE tube was used in the present example) was coupled to the corresponding cap and the other side was coupled to the ejection portion 20, and the inside of the supply was primed with the corresponding solution.

Thereafter, the hardening liquid (3 kPa) and the alginate-mixed solution (30 kPa) started to be ejected in this order, the pressurization liquid was ejected under the conditions described in Table 2 below, and the liquids were collected in a beaker containing an aqueous solution with the same component as the hardening liquid.

**[Table 2]**

| No. | Set Pressure (kPa) | Set Pressurization Time (ms) | Set Pressurization Intervals (ms) |
|---|---|---|---|
| 1 | 100 | 10 | 900 |
| 2 | 100 | 1 | 900 |

### [Example 2]

### (Case of Using Different Compositions for Hardening Liquid and Pressurization Liquid)

A 3-mass% sodium alginate aqueous solution was prepared. Thereafter, a blue dextran-containing aqueous solution was prepared and mixed with an equal volume of the sodium alginate aqueous solution to generate an alginate-mixed solution as a main liquid. Thereafter, a 100-mM calcium chloride aqueous solution was prepared as a hardening liquid. Also, saline was employed as a pressurization liquid.

The main liquid, the pressurization liquid, and the hardening liquid were filled into the main liquid storage portion 10 and the liquid tanks 31 and 32, respectively, and corresponding caps were coupled thereto. One side of each of the supply tubes 42, 53, and 54 (a silicon tube or a PTFE tube was used) was coupled to the corresponding cap and the other side was coupled to the ejection portion 20, and the inside of the supply was primed with the corresponding solution.

Thereafter, the hardening liquid (25 mL/min) and the main liquid (4 mL/min) started to be ejected in this order, the pressurization liquid was ejected under the conditions described in Table 3 below, and the liquids were collected in a beaker containing an aqueous solution with the same component as the hardening liquid.

**[Table 3]**

| No. | Composition of Pressurization Liquid | Set Pressure (kPa) | Set Pressurization Time (ms) | Set Pressurization Intervals (ms) |
|---|---|---|---|---|
| 1 | 100-mM calcium chloride aqueous solution | 20 | 10 | 900 |
| 2 | 100-mM calcium chloride aqueous solution | 16 | 1 | 500 |
| 3 | Saline for injection | 16 | 1 | 500 |

### [Example 3]

### (Case of Encapsulating Cells in Main Liquid)

A 3-mass% sodium alginate aqueous solution was prepared. Thereafter, a cell suspension (1×10^8 cells /mL) was prepared and mixed with an equal volume of the sodium alginate aqueous solution to generate an alginate-mixed solution as a main liquid. Thereafter, a 20-mM barium chloride aqueous solution containing 0.9% sodium chloride was prepared as a hardening liquid and a pressurization liquid. The main liquid, the pressurization liquid, and the hardening liquid were filled into respective bottles (the main liquid storage portion 10 and the liquid tanks 31 and 32) and corresponding caps were coupled thereto.

One side of each of the supply tubes 42, 53, and 54 (a silicon tube or a PTFE tube was used in the present example) was coupled to the corresponding cap and the other side was coupled to the ejection portion 20, and the inside of the supply was primed with the corresponding solution.

Thereafter, the hardening liquid (12 mL/min) and the alginate-mixed solution (2 mL/min) started to be ejected in this order, the pressurization liquid was ejected under the conditions described in Table 4 below, and the liquids were collected in a beaker containing an aqueous solution with the same component as the hardening liquid.

**[Table 4]**

| No. | Set Pressure (kPa) | Set Pressurization Time (ms) | Set Pressurization Intervals (ms) |
|---|---|---|---|
| 1 | 10 | 50 | 150 |

In the three examples described above, different solutions were used as the pressurization liquids. Still, in each example, a gel fiber was successfully generated and cut in a predetermined length. Note that conditions such as the pressures, times, and pressurization intervals can be set to various values according to the type of the gel fiber, as mentioned above, in any of the examples.

### <Modification>

In the above configuration, various types and forms of pressurization devices can be employed as the pumps 41, 51, and 52. For example, a pump of a type that supplies air into a liquid tank to pressurize the inside of the liquid tank and thereby pressurize the liquid stored therein may be used, or a device that directly pressurizes the liquid can be used. Specific examples of the devices include, for instance, pneumatic pumps, pulseless pumps, diaphragm pumps, centrifugal pumps, and so on.

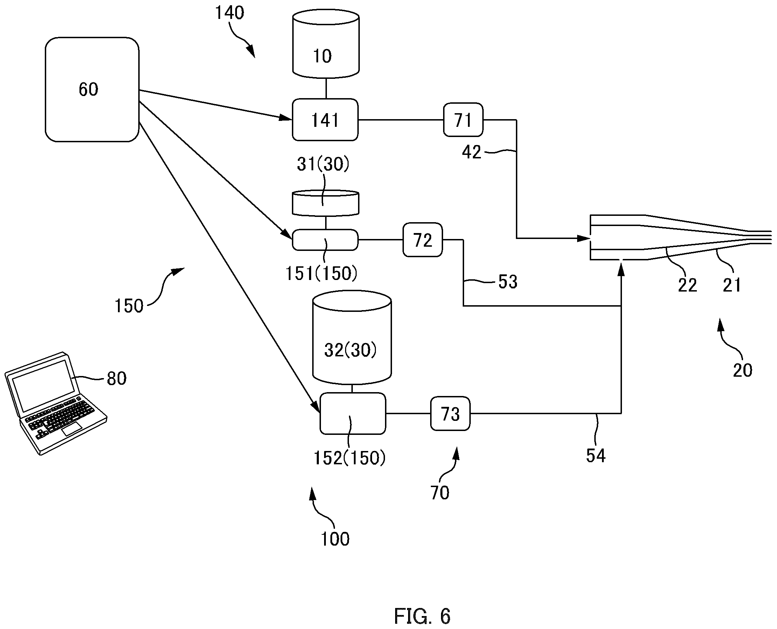

Also, the positions where the devices such as the pumps apply pressures to the liquids are not limited to the insides of the liquid tanks. For example, as illustrated in a gel fiber manufacturing apparatus 100 according to a modification in Fig. 6, a configuration in which pressurization devices are disposed on the supply tubes 42, 53, and 54 may be employed.

Specifically, the gel fiber manufacturing apparatus 100 includes a main liquid supply portion 140 and a sheath fluid supply portion 150 in addition to the main liquid storage portion 10, the ejection portion 20, the sheath fluid storage portion 30, the power output unit 60, the measurement device 70, and the controller 80. Note that similar components to those in the embodiment are denoted by the same reference signs, and description thereof is omitted.

A pump 141 in the main liquid supply portion 140 is mounted to the supply tube 42 coupling the main liquid storage portion 10 and the nozzle 22. The pump 141 pressurizes the main liquid inside the supply tube 42 to send the main liquid toward the nozzle 22.

Also, the sheath fluid supply portion 150 includes pumps 151 and 152. The pump 151 is mounted to the supply tube 53 coupling the liquid tank 31 and the supply tube 54. The pump 152 is mounted to the supply tube 54 coupling the liquid tank 32 and the nozzle 21.

The pump 151 pressurizes the pressurization liquid inside the supply tube 53 to send the pressurization liquid toward the supply tube 54. Also, the pump 152 pressurizes the hardening liquid inside the supply tube 54 to send the hardening liquid toward the nozzle 21.

In the above configuration too, the pumps 141, 151, and 152 pressurize the main liquid, the pressurization liquid, and the hardening liquid such that the gel fiber manufacturing apparatus 100 can generate and cut a gel fiber, as in the embodiment.

Note that the constant pressure P applied to the hardening liquid from the pumps 52 and 152 in the embodiment and the modification does not mean a pressure that is strictly constant, and the pressure is allowed to vary as long as the pressure can eject the sheath fluid from the nozzle 21 and form a gel fiber. Likewise, the pressure applied to the main liquid from the pumps 41 and 141 does not need to be a strictly constant pressure, and the pressure is allowed to vary as long as the pressure can eject the main liquid from the nozzle 22 and form a gel fiber.

### (Advantageous Effect)

In the above embodiments, aspects as below are disclosed.

(Aspect 1) In the above embodiments, the gel fiber manufacturing apparatuses 1 and 100 include: the nozzle 22 (corresponding to a first ejection portion) configured to eject a main liquid containing a core liquid; the nozzle 21 (corresponding to a second ejection portion) provided so as to surround a radially outer side of the first ejection portion and configured to eject a sheath fluid that hardens the core liquid; and the sheath fluid supply portions 50 and 150 configured to alternately apply a constant pressure and a high pressure being a higher pressure than the constant pressure to the sheath fluid to supply the sheath fluid to the nozzle 21.

With the above configuration, it is possible to manufacture a gel fiber and cut it in a target length without making a rough cut surface. Cutting a gel fiber with a cutting blade as in the conventional practice results in a failure to form a clear cut surface since the gel fiber is soft. Also, in the case of manufacturing a cell fiber, the cutting may damage contained cells. However, with the configuration, it is possible to neatly cut a gel fiber without making a rough cut surface or damaging substances contained in the gel fiber.

(Aspect 2) In aspect 1, the sheath fluid supply portion 50 and 150 apply the high pressure in a pulse waveform to the sheath fluid.

With the above configuration, applying the high pressure in a pulse waveform makes it possible to neatly cut a gel fiber.

(Aspect 3) In aspect 1 or 2, the gel fiber manufacturing apparatuses 1 and 100 include the main liquid supply portions 40 and 140 configured to supply the main liquid at a constant pressure to the first ejection portion.

With the main liquid supply portions 40 and 140 applying a constant pressure to the main liquid, it is possible to manufacture a gel fiber at a constant rate. This in turn makes it possible to cut a gel fiber in a target length.

(Aspect 4) In any of aspects 1 to 3, a combination of the core liquid and the sheath fluid is any of: alginic acid and/or a chemically modified alginic acid, and a polyvalent metal ion; a fibrin monomer and/or a chemically modified fibrin monomer, and a calcium ion; a collagen and/or a chemically modified collagen, and warm water; a carrageenan and/or a chemically modified carrageenan, and a calcium or potassium metal ion; an acrylic acid-based synthetic gel and a sodium ion; and a chitosan salt and/or a chemically modified chitosan salt, and an alkaline solution. Also, any of the components contained in the core liquid or the main liquid may form a salt, unless specifically stated otherwise.

(Aspect 5) The core liquid in the combination of the core liquid and the sheath fluid in aspect 4 further contains, as a composition, at least one of: alginic acid and/or a chemically modified alginic acid; a fibrin monomer and/or a chemically modified fibrin monomer; a collagen and/or a chemically modified collagen; a carrageenan and/or a chemically modified carrageenan; an acrylic acid-based synthetic gel; and a chitosan salt and/or a chemically modified chitosan salt.

Using the above-described core liquid and the sheath fluid enables the gel fiber manufacturing apparatuses 1 and 100 to manufacture and neatly cut a gel fiber.

(Aspect 6) In any of aspects 1 to 5, the first ejection portion has the nozzle 22 (corresponding to a main liquid nozzle) configured to eject the main liquid, and the second ejection portion has the nozzle 21 (corresponding to a sheath fluid nozzle) surrounding a radially outer side of the main liquid nozzle and configured to eject the sheath fluid.

With the above configuration, the gel fiber manufacturing apparatuses 1 and 100 can eject the main liquid from the single nozzle 22 to manufacture and neatly cut a polycationic gel fiber.

(aspect 7) In any of aspects 1 to 6, the main liquid contains one or both of a cell and a compound.

With the above configuration, the gel fiber manufacturing apparatuses 1 and 100 can manufacture a gel fiber that is a cell-containing gel fiber and manufacture a gel fiber containing a chemical.
In either case, the gel fiber manufacturing apparatuses 1 and 100 can manufacture and neatly cut the gel fiber.

In each of the above configurations, a high pressure is applied to the sheath fluid through the pressurization liquid. Alternatively, a configuration that does not use the pressurization liquid may be employed. For example, the sheath fluid storage portion 30 may be a single liquid tank that stores the sheath fluid. In this case, the sheath fluid can be supplied to the ejection portion 20 through the supply tube 54 by applying the pressure time history illustrated in Fig. 3(c) directly to the sheath fluid.

### [Reference Signs List]

1, 100 gel fiber manufacturing apparatus
10 main liquid storage portion
20 ejection portion
30 sheath fluid storage portion
40, 140 main liquid supply portion
50, 150 sheath fluid supply portion
60 power output unit
70 measurement device
80 controller

## Claims

1. A gel fiber manufacturing apparatus comprising:
a first ejection portion configured to eject a main liquid containing a core liquid;
a second ejection portion provided to surround a radially outer side of the first ejection portion and configured to eject a sheath fluid that hardens the core liquid; and
a sheath fluid supply portion configured to alternately apply to the sheath fluid a constant pressure and a high pressure to supply the sheath fluid to the second ejection portion, the high pressure being higher than the constant pressure.

2. The gel fiber manufacturing apparatus according to claim 1, wherein the sheath fluid supply portion applies the high pressure in a pulse waveform to the sheath fluid.

3. The gel fiber manufacturing apparatus according to claim 1 or 2, further comprising a main liquid supply portion configured to supply the main liquid at a pressure constantly to the first ejection portion.

4. The gel fiber manufacturing apparatus according to claim 1 or 2, wherein a combination of a composition contained in the core liquid and a composition contained in the sheath fluid is any of
alginic acid and/or a chemically modified alginic acid, and a polyvalent metal ion,
a fibrin monomer and/or a chemically modified fibrin monomer, and a calcium ion,
a collagen and/or a chemically modified collagen, and warm water,
a carrageenan and/or a chemically modified carrageenan, and a calcium or potassium ion,
an acrylic acid-based synthetic gel and a sodium ion, and
a chitosan salt and/or a chemically modified chitosan salt, and an alkaline solution.

5. The gel fiber manufacturing apparatus according to claim 4, wherein the core liquid in the combination further contains, as a composition, at least one of
alginic acid and/or a chemically modified alginic acid,
a fibrin monomer and/or a chemically modified fibrin monomer,
a collagen and/or a chemically modified collagen,
a carrageenan and/or a chemically modified carrageenan,
an acrylic acid-based synthetic gel, and
a chitosan salt and/or a chemically modified chitosan salt.

6. The gel fiber manufacturing apparatus according to claim 1 or 2, wherein
the first ejection portion has a main liquid nozzle configured to eject the main liquid, and
the second ejection portion has a sheath fluid nozzle surrounding a radially outer side of the main liquid nozzle and configured to eject the sheath fluid.

7. The gel fiber manufacturing apparatus according to claim 1 or 2, wherein the main liquid contains a cell and/or a compound.

8. A gel fiber manufacturing method comprising:
ejecting a main liquid containing a core liquid; and
ejecting a sheath fluid such that the sheath fluid surrounds a radially outer side of the ejected main liquid, the sheath fluid being a fluid which hardens the core liquid; and
alternately applying to the sheath fluid a constant pressure and a high pressure which is higher than the constant pressure.
